# EUROPÄISCHE PATENTANMELDUNG

(11) **EP 2 246 092 A1**
(43) Veröffentlichungstag der Anmeldung: **03.11.2010**
(21) Anmeldenummer: 10157044.8
(22) Anmeldetag: 19.03.2010
(51) Int. Cl.: A61N 1/05, H01B 7/04, A61N 1/04, A61B 5/04

(54) **Verfahren zur Herstellung einer Elektrodenleitung**

(30) Priorität: 29.04.2009 DE 102009002707
(71) Anmelder: Biotronik CRM Patent AG, 6341 Baar (CH)
(72) Erfinder: Bartels, Klaus, 10115, Berlin (DE); Günther, Thomas, 14552, Michendorf OT Wilhelmshorst (DE); Landgraf, Tassilo, 12277, Berlin (DE); Fründt, Carsten, 13057, Berlin (DE)
(74) Vertreter: Lindner-Vogt, Karin L.

(57) **Zusammenfassung**

Die Erfindung betrifft ein Herstellverfahren für eine Elektrodenelement (10, 20) und eine implantierbare Elektrodenleitung (100) welches die Schritte Herstellen des Elektrodenelements durch Formen der Elektrode (11, 21) aus dem Seil der Zuleitung (12, 22), Formen des Elektrodenleitungskörpers (110), und Montieren des Steckers und des Elektrodenelementes an den Elektrodenleitungskörper umfasst.

## Beschreibung

Der Gegenstand der Patentanmeldung betrifft Verfahren zur Herstellung einer Elektrodenleitung, welches ein elektrisch aktives Elektrodenelement mit einer Elektrode mit einer nach außen weisenden elektrisch aktiven Elektrodenfläche und einer lang gestreckte elektrische Zuleitung umfasst, wobei das Elektrodenelement an seinem proximalen Ende eine elektrische Verbindung zu einem elektrisch aktiven Implantat herstellen kann und welche durchgehend als elektrisch leitendes Seil ausgebildet ist.

Solche Elektrodenelemente im weitestgehenden Sinne sind bereits aus dem Stand der Technik bekannt. So offenbart bereits die US 7,174,220 B1 ein solches Elektrodenelement als Kombination einer Elektrode und einer lang gestreckten Zuleitung, die die Elektrode elektrisch mit einem Elektrodenstecker am proximalen Ende einer Elektrodenleitung verbindet. Dabei ist die Zuleitung nicht die dem Fachmann bekannte übliche helixförmige Zuleitung, sondern eine lang gestreckte, longitudinale Zuleitung, die in möglichst lang gestreckter Richtung eine Verbindung zwischen Elektrode und Stecker herstellt. Dies verbessert die Flexibilität und Biegbarkeit, die eine implantierbare Elektrodenleitung, für die ein solches Elektrodenelement geeignet ist, benötigt. Weiterhin wird durch diesen lang gestreckten Aufbau der Durchmesser einer Elektrodenleitung verringert.

Aus der genannten Schrift ist bekannt, diese Zuleitung aus einem Seil zu fertigen. Ein Seil im Sinne der Erfindung ist ein aus zusammengedrehten Einzeldrähten bestehendes längliches, flexibles und elastisches Element, in diesem Fall zur Übertragung von Energie. Das Seil wird aus einer oder mehreren Litzen (typischerweise 1, 3, 7 oder 12 Litzen) mittels "Verseilen" gefertigt, die in einem separaten Fertigungsschritt aus Einzeldrähten (typischerweise 3, 7, 19 Einzeldrähte) mittels "Verlitzen" hergestellt werden. Die Einzeldrähte können dabei aus Vollmaterial oder aus so genannten Mantel- oder DFT-Drähten (DFT = Drawn Filled Tube) bestehen, wobei Letztere Drähte mit einem Kernmaterial und einem vom Kernmaterial verschiedenen Mantelmaterial ist. Als Materialien für den Volldraht und das Kernmaterial eignen sich bevorzugt biokompatible und biostabile Metalle wie beispielsweise Platin-Iridium Legierungen, Tantal, Niob, Zirkonium und Molybdän einschließlich deren Legierungen. Zusätzlich eignen sich bei Manteldrähten generell besonders für das Mantelmaterial Platin-Iridium-Legierungen und für das Kernmaterial Kobalt-Chrom-Basislegierungen wie beispielsweise MP35N.
Bezüglich des Manteldrahtes betragen die Verhältniszahlen der Querschnittsflächen von Kern zu Mantel 25 - 95%.

Eine Elektrode im Sinne der Erfindung ist ein elektrisch aktives Element, welches geeignet ist, elektrische Energie oder elektrische Impulse (wie Stimulationsimpulse oder Defibrillationsschocks) abzugeben oder aber um Signale aus der Umgebung der Elektrode aufzunehmen und weiterzuleiten. Solche Stimulations- und Wahrnehmungselektroden bestehen im Stand der Technik wie der US 7,174,220 B1 aus einer starren Metallhülse (beispielsweise aus Rohrabschnitten), welche mittels Schweißen (Laserschweißen, Widerstandsschweißen), Löten oder Crimpen mit der Zuleitung elektrisch verbunden ist. In einer anderen Ausführungsform im Stand der Technik besteht eine Elektrode, vorzugsweise eine Schockelektrode, aus einem frei gelegten Wendelabschnitt einer wendelförmigen Zuleitung. Ein solcher Aufbau hat einen großen Durchmesser und geringe Flexibilität zur Folge.

Damit eine Elektrode Energie oder Impulse abgeben kann oder Körpersignale wie beispielsweise Temperatur, Impedanz, Herz- oder Hirnsignale aufnehmen kann, weist sie eine nach außen - also in Richtung der Umgebung der Elektrode - weisende elektrisch aktive Fläche auf, welche im Einbauzustand in eine Elektrodenleitung gegenüber der Umgebung unisoliert ist.

Das beschriebene Elektrodenelement aus dem Stand der Technik hat jedoch den besonderen Nachteil, dass die Zuleitung bzw. der Übergang zwischen Zuleitung und Elektrode eine erhöhte Bruchanfälligkeit aufweist. Am Implantationsort einer mit einem Elektrodenelement bestückten Elektrodenleitung wirken hohe und immerwährende Biegebelastungen, die durch Körperbewegung oder Herzschlag oder aber unterschiedliche Herzdrücke entstehen. Bei solchen Biegebelastungen ist der Übergang von einer flexiblen Zuleitung zu der starren Elektrode von besonderem Nachteil, da sich an dieser Übergangsstelle bei Biegebelastungen hohe Kräfte bilden. Durch die starren, fast schön spröden gefügten Übergangsstellen entsteht bei solchen Elektrodenelementen aus dem Stand der Technik ein Zuverlässigkeitsproblem, was sich an diesen Stellen in Brüchen und Wackelkontakten widerspiegelt. Solche Defekte sind für den Patienten lebensbedrohend und nicht reparabel. Folge ist der Austausch der kompletten Elektrodenleitung, welcher ebenfalls sehr riskant ist.
Des Weiteren weisen namentlich Crimp-Verbindungen zwischen Zuleitung und Elektrode eines Elektrodenlementes aus dem Stand der Technik zur sicheren Verbindung als Gegenlager notwendige zusätzliche Verbindungshülsen bzw. -stifte auf. Dadurch wird eine Durchmesservergrößerung bewirkt, was sich insofern nachteilig auswirkt, dass der Blutfluss von intravaskulär implantierbaren Elektroden gestört wird beziehungsweise ein Einsatz in einem Herzkranzgefäß zur Stimulation der linken Herzhälfte nicht mehr möglich ist.

DE 197 58 368 A1 offenbart ein solches elektrisch aktives Elektrodenelement für eine medizinische Elektrodenleitung, welches eine Elektrode mit einer nach außen weisenden elektrisch aktiven Elektrodenfläche und eine lang gestreckte elektrische Zuleitung, welche an seinem proximalen Ende eine elektrische Verbindung zu einem elektrisch aktiven medizinischen Gerät herstellen kann und welche durchgehend als elektrisch leitendes Seil ausgebildet ist, aufweist. Dieses Elektrodenelement zeichnet sich dadurch aus, dass das Seil der Zuleitung an seinem distalen Ende die Elektrode bildet.

Ein elektrisch medizinisches Gerät kann sowohl ein externes Gerät wie ein externe Defibrillator oder andere Geräte zum Stimulieren eines menschlichen oder tierischen Körpers oder zum diagnostischen Messen von Körpersignalen sein. Besonders bevorzugt sind elektrisch aktive Implantate wie Herzschrittmacher, Kardioverter/Defibrillatoren oder Nervenstimulatoren. Auch sind andere Implantate umfasst, welche beispielsweise nur dazu dienen, intrakorporal Signale zu sammeln und an einen außerhalb des Körpers befindlichen Empfänger zu senden. Ebenfalls können Implantate umfasst sein, die mithilfe von Elektrodenleitungen elektrische Signale aufnehmen, aber keine elektrotherapeutische Stimulation erzeugen, beispielsweise durch Medikamentenabgabe.

Das genannteElektrodenelement und die Elektrodenleitung zeichnen sich durch eine hervorragende Bruchsicherheit aus, da ein Materialstrang durchgehend die Zuleitung und die Elektrode bildet. Es entsteht also keine für Brüche unter Wechselbelastung anfällige Füge- oder Verbindungsstelle und auch kein Übergang von einem flexiblen zu einem starren Abschnitt.

Weiterhin zeichnet sich das genannte Elektrodenelement dadurch aus, dass erstmals eine komplett flexible Elektrode für geringe Durchmesser und kleine Stimulationsflächen vorgesehen werden können.

Zudem hat sich vorteilhafterweise herausgestellt, dass Elektrodenelemente, bei denen die lang gestreckte Zuleitung und die Elektrode aus einem Materialstrang bestehen, eine hohe Spannungsfestigkeit aufweisen, wodurch ein flexibler Einsatz sowohl bei Defibrillationselektroden als auch Stimulationselektroden vorgesehen werden kann.

Das distale Ende des Zuleitungsseiles kann rohr- oder zylinderartig geformt sein und bildet so die Elektrode mit der nach außen weisenden elektrisch aktiven Elektrodenfläche, wobei vorzugsweise die Längsachse der rohr- oder zylinderartig geformten Elektrode in Verlängerung zur lang gestreckten Zuleitung liegt.

Bevorzugt ist die rohrförmige Elektrode helixförmig ausgebildet, indem das Seil um die Längsachse herum aufgewickelt oder aufgespult ist.

Gemäß einer weiteren Alternative des Elektrodenelements ist das distale Ende des Zuleitungsseiles mäanderförmig geformt und die Elektrode als eine ebene Fläche mit zwei rechtwinklig zueinander liegenden gedachten Außenkanten ausgestaltet, wodurch die nach außen weisende elektrisch aktive Elektrodenfläche gebildet ist. Die Elektrode liegt dabei vorzugsweise in Verlängerung der Zuleitung, wobei besonders bevorzugt die flache Elektrode rohr- oder zylinderartig geformt ist.

Beide Alternativen zeugen von der flexiblen Einsetzbarkeit solcher Elektrodenelemente. Zum einen können sie als implantierbare Elektrodenleitung intrakardial, epikardial, im Koronarsinus oder zur Stimulation von Nerven eingesetzt werden. Diese weisen über ihre komplette Länge einen runden Querschnitt auf. Zum anderen können die Elektrodenelemente auch in Patch-Elektrodenleitungen eingesetzt werden, welche eine besondere Ausgestaltung von epikardialen Elektrodenleitungen sind und welche eine großflächige Stimulation außerhalb des Herzens erlauben. Denkbar sind auch externe Anwendungen, beispielsweise als externe Defibrillationselektrodenleitungen oder aber Elektrodenleitungen, mit denen nicht invasiv Körpersignale gemessen werden können.

Bei beiden Alternativen bildet das Seil im Bereich der Elektrode eine Vielzahl helixförmiger oder nebeneinander liegender Gänge, welche vorzugsweise zumindest abschnittsweise verschweißt sind.

Durch diese Anordnung wird der elektrische Widerstand verringert, was auch weiter zu einer weiteren Erhöhung der Spannungsfestigkeit und Zuverlässigkeit führt.

Das Seil des Elektrodenelements ist aus Manteldrähten und/oder Drähten aus Vollmaterial gebildet, wobei der Kerndraht jedes einzelnen Manteldrahtes oder des einzelnen Drahtes aus Vollmaterial aus einem der Materialien ausgewählt aus Molybdän, Tantal, Niob, Zirkonium oder einer Platin-Iridium-Legierung besteht.

Die implantierbare Elektrodenleitung umfasst einen Elektrodenleitungskörper mit einem proximalen und einem distalen Ende, einen am proximalen Ende des Elektrodenleitungskörpers befindlichen Stecker zur festen elektrischen Kontaktierung mit einem elektrisch aktiven Implantat, und mindestens ein soeben genanntes Elektrodenelement.

Dadurch wird eine flexible und möglichst dünne Elektrodenleitung geschaffen, welche zuverlässig, bruchsicher und einfach herzustellen ist.

Durch den Einsatz des Elektrodenelements erhält die Elektrodenleitung dieselben positiven Eigenschaften wie hohe Bruchsicherheit, geringe Durchmesser des Elektrodenleitungskörpers und hohe Spannungsfestigkeit.

Das proximale Ende der Zuleitung des Elektrodenelements ist am proximalen Ende des Elektrodenleitungskörpers mit dem Stecker elektrisch leitend verbunden, wobei die Elektrode am distalen Ende und/oder im distalen Endbereich des Elektrodenleitungskörpers liegt.

Der distale Endbereich ist der Bereich, der sich an der Stelle im oder am Körper, Körpermedium und/oder Körpergewebe befindet, an dem Signale aufgenommen werden sollen und/oder an dem der Körper stimuliert werden soll.

Weiterhin weist der Elektrodenleitungskörper am distalen Ende oder im distalen Bereich mindestens eine Aussparung, vorzugsweise eine Ringnut oder Quernut auf, in der die Elektrode form- und lagestabil liegt, wobei die nach außen weisende elektrisch aktive Elektrodenfläche so in den Elektrodenleitungskörper eingepasst ist, dass diese mit der nach äußeren isolierten Oberfläche isodiametrisch oder isoplanar ist.

Durch diese Anordnung ist es möglich, eine Elektrodenleitung herzustellen, welche keine Absätze oder hervorstehenden, bei der Implantation und im Körper störenden Teile aufweist. Eine sichere und zielgerichtete Positionierung und ein sicherer Betrieb wird dadurch gewährleistet.

Auch kann die Zuleitung des Elektrodenelements innerhalb des Elektrodenleitungskörpers isoliert verlaufen und im Bereich seines distalen Endes aus dem Elektrodenleitungskörper herausgeführt werden, damit die Elektrode, vorzugsweise die nach außen weisende elektrisch aktive Elektrodenfläche, unisoliert Kontakt zu Körpermedium und/oder Körpergewebe hat.

Weiterhin kann in allen Ausführungsformen der Elektrodenleitung der Elektrodenleitungskörper flexibel und biegbar sein und vorzugsweise aus einem Kunststoff wie Silikon oder Polyurethan bestehen. Zusätzlich weist der Elektrodenleitungskörper der Elektrodenleitung mindestens eine erste Bohrung zur Aufnahme der Zuleitung und eine zweite Bohrung zur vorübergehenden Aufnahme eines Führungsdrahtes auf, wobei die erste Bohrung ein proximales Ende am oder im proximalen Ende des Elektrodenleitungskörpers und ein distales Ende in oder an einer ihr zugeordneten Aussparung aufweist.

Durch diese innovative Anordnung wird die Herstellung der Elektrodenleitung erleichtert. Die Herstellung mit wenigen Schritten ermöglicht eine hochautomatisierte und ökonomische Herstellung von sämtlichen implantierbaren Elektrodenleitungen. Weiterhin wird eine angepasste und flexible Produktion von verschiedenartigen implantierbaren Elektroden ermöglicht.

Der Patentanmeldung liegt die Aufgabe zugrunde, ein ökonomisches, flexibles und leichtes Herstellverfahren für solche Elektrodenleitungen mit dem genannten Elektrodenelement zu schaffen.

Diese Aufgabe wird durch den Gegenstand des Anspruchs 1 gelöst, indem das Herstellverfahren für das Elektrodenelement und die implantierbare Elektrodenleitung die folgenden Schritte umfasst:
- Herstellen des Elektrodenelements durch Formen der Elektrode aus dem Seil der Zuleitung
- Formen des Elektrodenleitungskörpers
- Montieren des Steckers und des Elektrodenelementes an den Elektrodenleitungskörper.

Vorzugsweise umfasst der Formungsschritt der Herstellung des Elektrodenelements das Wickeln oder Spulen oder mäanderförmiges Formen des distalen Endes des Seiles.

Weiter bevorzugt umfasst der Schritt zur Formung des Elektrodenleitungskörpers ein Spritzgussverfahren, wobei Aussparung und die erste und zweite Bohrung geformt werden.

Besonders bevorzugt umfasst der Montageschritt zusätzlich die folgenden Schritte:
- Einfädeln des proximalen Endes der Zuleitung in die erste Bohrung in oder an der zugeordneten Aussparung und Schieben der Zuleitung in Richtung des proximalen Endes des Elektrodenleitungskörpers, bis die Elektrode in der zugeordneten Aussparung zu liegen kommt,
- Kleben der Elektrode an den Elektrodenleitungskörper in der Aussparung,
- elektrisches Verbinden des proximalen Endes der Zuleitung mit dem Stecker und Befestigen des Steckers am Elektrodenleitungskörper.

Weitere Ausführungsmöglichkeiten sind in der Beschreibung oder den Zeichnungen aufgeführt.

Anhand der Figuren wird im Folgenden die Erfindung beschrieben. Es zeigen:
- Fig. 1a: eine Ausführungsform des Elektrodenelements
- Fig. 1b: eine Schnittdarstellung durch die Elektrode des Elektrodenelements aus Fig. 1a entlang der der Schnittlinie 1b
- Fig. 2a: eine weitere Ausführungsform des Elektrodenelements
- Fig. 2b: eine Schnittdarstellung durch die Elektrode des Elektrodenelements aus Fig. 2a entlang der Schnittlinie 2b
- Fig. 3: eine implantierbare Elektrodenleitung mit einem Elektrodenelement
- Fig. 4a: einen Elektrodenleitungskörper für eine Elektrodenleitung mit einem Elektrodenelement
- Fig. 4b: eine Schnittdarstellung durch die Aussparung des Elektrodenlei- tungskörpers entlang der Schnittlinie 4b
- Fig. 4c: eine Schnittdarstellung durch den Elektrodenleitungskörper entlang der Schnittlinie 4c
- Fig. 5a: einen Elektrodenleitungskörper für eine mehrpolige implantierbare Elektrodenleitung
- Fig. 5b bis Fig. 5d: Schnittdarstellungen des Elektrodenleitungskörpers für mehrpolige implantierbare Elektrodenleitungen entlang der Schnittlinien 5b bis 5d

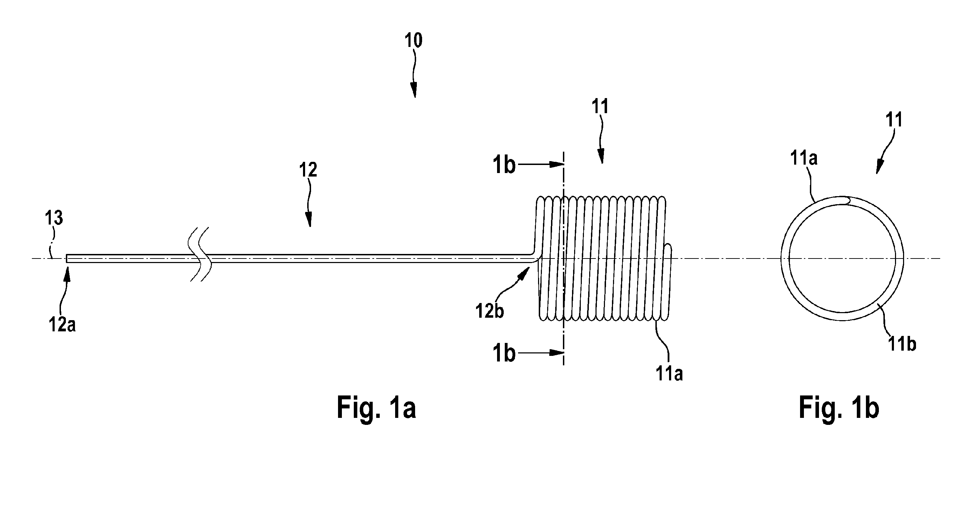

Fig. 1a und 1b zeigen ein Elektrodenelement 10 entsprechend einer ersten Ausführung mit einer Elektrode 11 und einer Zuleitung 12 und einen Schnitt durch die Elektrode 11. Die Zuleitung 12 besteht dabei aus einem Seil aus Volldraht- oder Manteldrahtlitzen oder - fasern der eingangs zitierten Art. Das distale Ende des Seiles bildet die Elektrode 11, indem in dieser Ausführung der Draht entlang einer zentral liegenden Längsachse 13 helixförmig aufgewickelt oder aufgespult ist. Die Zuleitung 12 liegt in Verlängerung dieser Längsachse 13 und kann entweder parallel zu dieser oder aber auf dieser Längsachse liegen. Die Elektrode 11 befindet sich somit unmittelbar distal des distalen Endes 12b der Zuleitung 12.
Die Elektrode 11 bildet weiterhin eine nach außen (von der Längsachse 13 entgegengesetzt) weisende elektrisch aktive Elektrodenfläche 11a, welche in einem eingebauten Zustand mit Körpermedium und/oder Körpergewebe in Kontakt steht. Durch die helixförmig aufgewickelte Art bildet das Seil Gänge 11b. Als Gang gilt dabei eine Umdrehung des Drahtes um die Längsachse 13 herum. Bei jeder weiteren Umdrehung wird ein weiterer Gang gebildet. Die Gänge 11b sind bevorzugt direkt distal folgenden oder proximal vorhergehenden Gang anliegend, können aber auch andersartig ausgebildet sein, beispielsweise indem ein mit Isolationsmaterial aufgefüllter Zwischenraum zwischen jedem Gang 11b besteht. Somit wird die Elektrodenoberfläche 11 a generell von einem Zwischenraum zwischen jedem Gang 11b unterbrochen.

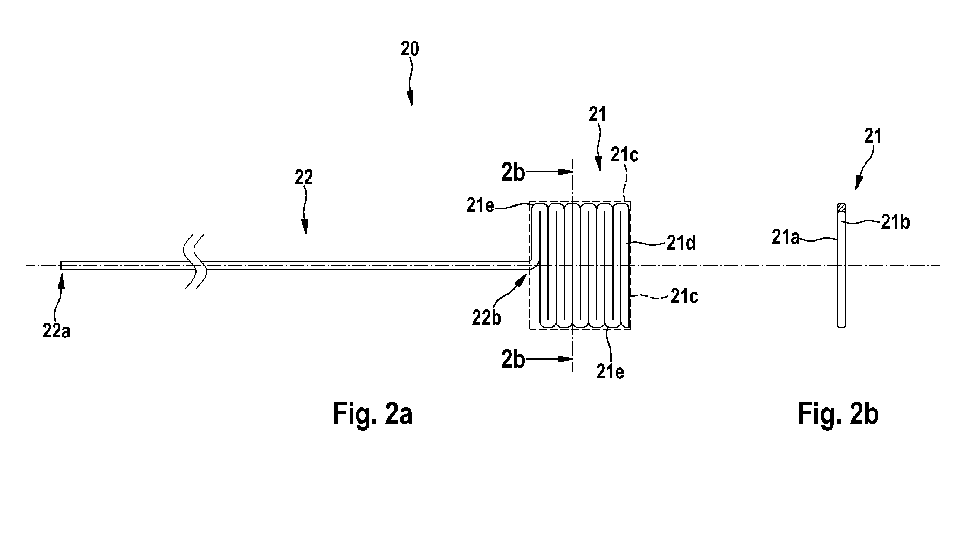

Eine weitere Ausführungsform des Elektrodenelements 20 ist in den Fig. 2a und 2b gezeigt. Dort wird die Elektrode 21 nicht durch helixförmiges Aufwickeln oder Spulen des Seiles gebildet, sondern durch eine mäanderförmige Zick-Zackformung bestehend aus geradlinigen Abschnitten 21d, deren entgegen gesetzten Enden einerseits mit dem distal vorhergehenden und andererseits mit dem proximal nachfolgenden geradlinigen Abschnitt 21 d durch entgegen gesetzte gebogene Abschnitte 21e verbunden sind. Es entsteht somit im Gegensatz zur vorherigen Ausführungsform eine ebene nach außen weisende Elektrodenfläche 21a mit zwei rechtwinklig zueinander liegenden, gedachten Außenkanten 21c. Diese Anordnung bildet ebenfalls Gänge 21b, welche durch einen geradlinigen Abschnitt von einem gebogenen Abschnitt am einen Ende bis zum gebogenen Abschnitt am anderen Ende des geradlinigen Abschnitts erstreckt.

Die Elektrode 21 befindet sich in Verlängerung zur lang gestreckten Zuleitung 22 an dessen distalem Ende 22b und wird ebenfalls durch das distale Ende des Zuleitungsseils gebildet.

In der genannten Konfiguration kann dieses Elektrodenelement beispielsweise Einsatz in einer so genannten Patch-Elektrodenleitung oder aber in einer externen, auf den Körper auflegbaren Wahrnehmungselektrodenleitung eingesetzt werden. Bevorzugt ist die ebene, flache Elektrode 21 jedoch rohr- oder zylinderförmig entlang einer Längsachse geformt, wobei die lang gestreckte Zuleitung 22 auf oder parallel dieser Längsachse liegt. So entsteht eine einfach herstellbare Konfiguration, welche beispielsweise in einer implantierbaren Elektrodenleitung eingesetzt werden kann.

Bei beiden Ausführungen können einzelne, mehrere oder alle Gänge 11b und 21b beispielsweise durch Laser- oder Widerstandsschweißen miteinander verschweißt sein. Dies kann beispielsweise ein abwechselnd paarweises Verschweißen von Gängen 11b an im Umfangsrichtung entgegengesetzten Stellen erfolgen, um die Flexibilität nicht zu vermindern.

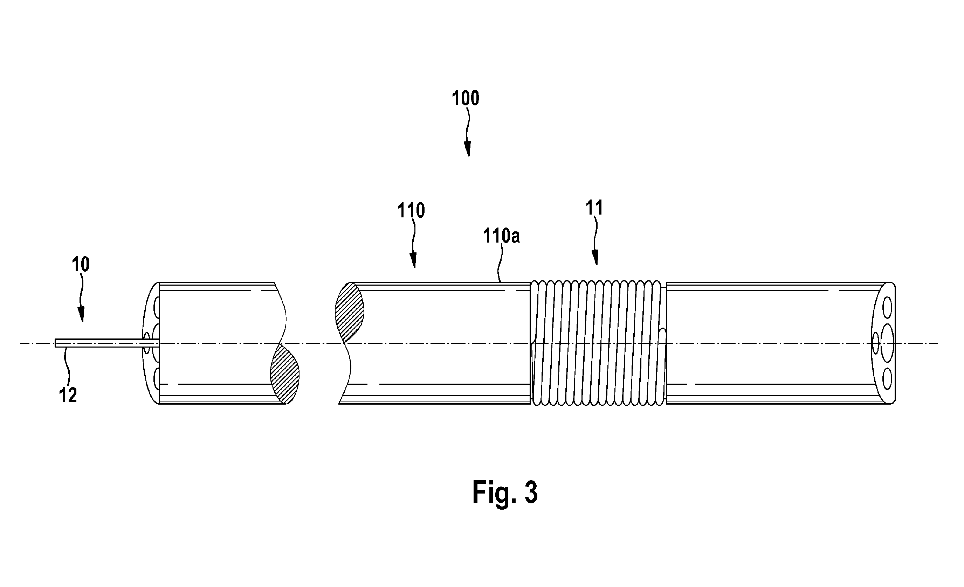

Fig. 3 zeigt den Abschnitt einer implantierbaren Elektrodenleitung 100 mit einem Elektrodenelement 10. Das Elektrodenelement 10 ist in einen Elektrodenleitungskörper 110 eingebaut, so dass die Elektrode 11 isodiametrisch zur äußeren isolierten Oberfläche 110a des Elektrodenleitungskörpers liegt. Entlang der Längsachse des Elektrodenleitungskörpers 110 verläuft die Zuleitung 12, um mit einem am distalen Ende des Elektrodenleitungskörpers befindlichen und angebauten Stecker elektrisch verbunden zu sein. Dieser Stecker entspricht einer der Normen IS-1, DF-1, IS-4, mit Hilfe dessen eine elektrische Verbindung zu einem elektrisch aktiven Implantat der eingangs genannten Art hergestellt werden kann.

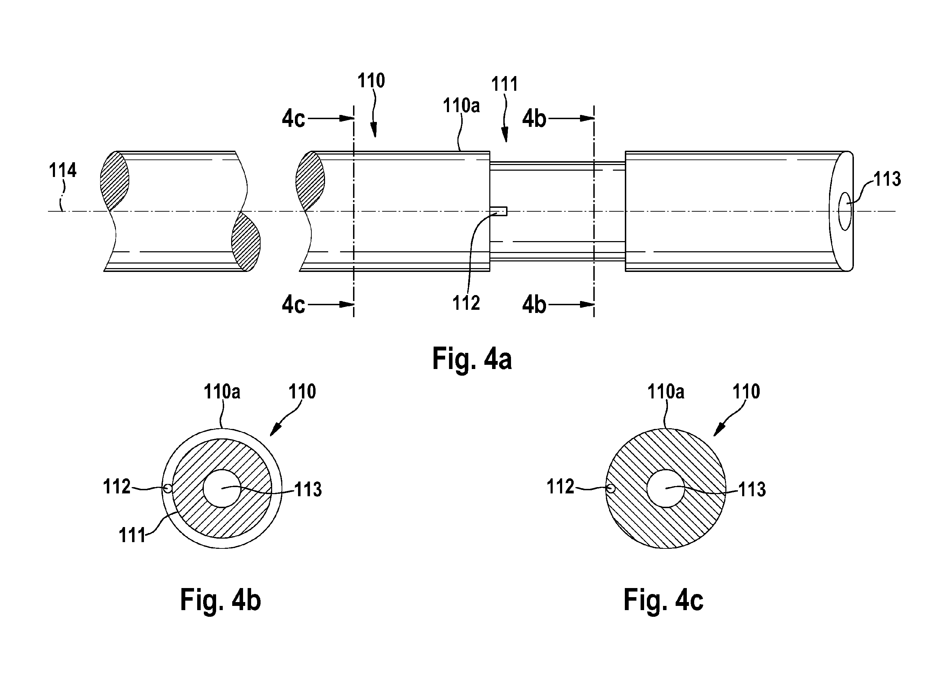

Im Detail wird der Elektrodenleitungskörper 110 in den Fig. 4a bis 4c dargestellt. Der Elektrodenleitungskörper weist am oder in der Nähe seines distalen, dem Stecker abgewandten Endes eine Aussparung 111 in Form einer Ringnut auf, welche eine radiale Tiefe aufweist, die der Dicke des Seils und damit der Elektrode 11 entspricht. Das heißt, der Durchmesser der nach außen weisenden elektrisch aktiven Fläche 11a entspricht dem größten Durchmesser des Elektrodenleitungskörpers 110 an seiner Stelle des größten Durchmessers an der äußeren isolierten Oberfläche, während der Innendurchmesser der Elektrode 11 dem Außendurchmesser der Aussparung 111 entspricht.

Mit der Aussparung 111 in Kontakt steht eine erste Bohrung 112 mit ihrem distalen Ende, wobei diese Bohrung parallel zur Längsachse 114 bis zum proximalen Ende des Elektrodenleitungskörpers 110 verläuft. Diese Bohrung 112 nimmt im zusammengesetzten Zustand der implantierbaren Elektrodenleitung 100 die Zuleitung 12 auf, welche an deren distalen Ende 12b aus der Bohrung des Elektrodenleitungskörpers 110 in die Aussparung 111 herausgeführt ist. Sowohl die Elektrode 11 als auch die Zuleitung 12 können in die Aussparung 111 bzw. in die Bohrung 112 eingeklebt sein.

Des Weiteren kann ein solcher Elektrodenleitungskörper 110 zusätzlich eine zweite Bohrung 113 aufweisen, anhand derer die implantierbare Elektrodenleitung 100 mit Hilfe eines Führungsdrahtes geführt werden kann. Diese liegt üblicherweise auf der Längsachse 114. Zusätzlich kann die Elektrode die üblichen, aus dem Stand der Technik bekannten aktiven und passiven Befestigungen aufweisen.

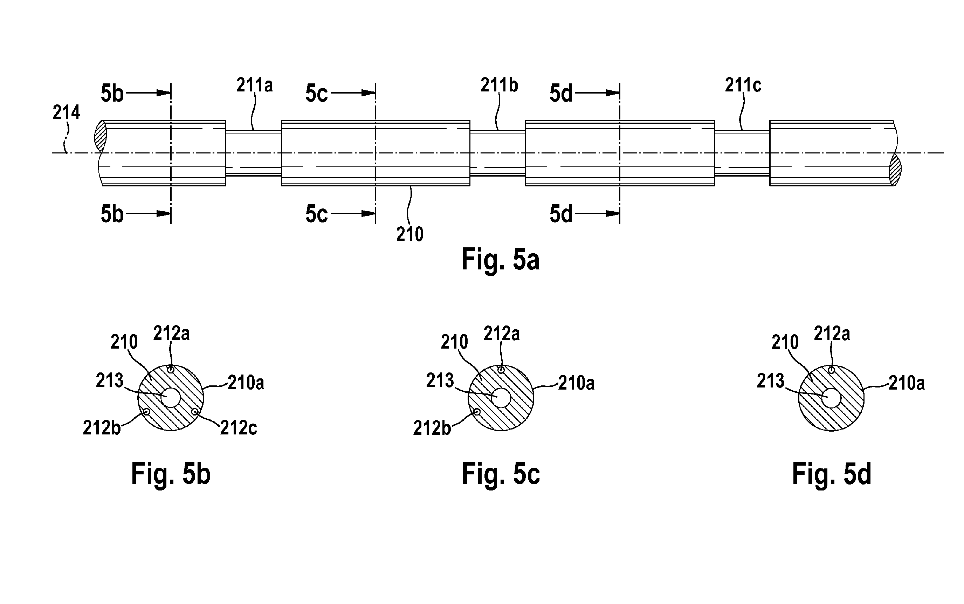

In einer weiteren Ausgestaltung der implantierbaren Elektrodenleitung kann diese mehrpolig, das heißt mit zwei oder mehreren Elektroden ausgeführt sein. Dazu kann beispielsweise bei einer drei-poligen Elektrodenleitung der Elektrodenleitungskörper 210 den in Fig. 5a bis 5d gezeigten Aufbau haben. Er weist dann drei in Fig. 4a bis 4c beschriebene Kombinationen aus Aussparungen 211a, 211b und 211 c auf, denen mit jeweils einer dieser Aussparungen in Verbindung Bohrungen 212a, 212b und 212c zugeordnet sind. Jede dieser Bohrung erstreckt sich von einem distalen Ende, welches sich in oder an den zugeordneten Aussparungen befindet, entlang der Längsachse 214 des Elektrodenleitungskörpers 210 bis zum proximalen Ende des Elektrodenleitungskörpers, an dem ein bekannter Stecker zur elektrischen Verbindung mit einem Implantat angebracht ist. Da die Aussparungen 211a, 211b und 211c, die sich alle im distalen Bereich in der Nähe des distalen Endes befinden, an unterschiedlichen Positionen entlang der Längsachse des Elektrodenleitungskörpers 210 befinden, sind die Bohrungen 212a, 212b und 212c von unterschiedlicher Länge. Damit die notwendige Isolierung gegenüber den anderen Zuleitungen 12 gegeben ist, sind die Bohrungen wie in fig. 5b bis 5d dargestellt in Umfangsrichtung um die Längsachse 214 oder um die Bohrung 213 herum vorteilhafterweise regelmäßig verteilt. Mit diesem Aufbau sind auch implantierbare Elektrodenleitungen mit noch mehr Elektroden möglich.

## Patentansprüche

**1.** Herstellverfahren für eine Elektrodenelement (10, 20) für eine medizinische Elektrodenleitung,
wobei das Elektrodenelement eine Elektrode (11, 12) mit einer nach außen weisenden elektrisch aktiven Elektrodenfläche (11a, 21a), und eine lang gestreckte elektrische Zuleitung (12, 22), welche an seinem proximalen Ende (12a, 22a) eine elektrische Verbindung zu einem elektrisch aktiven medizinischen Gerät herstellen kann und welche durchgehend als elektrisch leitendes Seil ausgebildet ist und bei der das Seil an seinem distalen Ende die Elektrode bildet;
und wobei Elektrodenleitung (100) neben dem Elektrodenelement (10, 20) einen Elektrodenleitungskörper (110) mit einem proximalen und einem distalen Ende und einen am proximalen Ende des Elektrodenleitungskörpers befindlichen Stecker zur festen elektrischen Kontaktierung mit einem elektrisch aktiven Implantat, umfasst; wobei das Verfahren folgende Schritte umfasst:
- Herstellen des Elektrodenelements durch Formen der Elektrode (11,21) aus dem Seil der Zuleitung (12, 22)
- Formen des Elektrodenleitungskörpers (110)
- Montieren des Steckers und des Elektrodenelementes an den Elektrodenleitungskörper.

**2.** Verfahren nach Anspruch 1, wobei der Formungsschritt der Herstellung des Elektrodenelements (10, 20) Wickeln oder Spulen oder mäanderförmiges Formen des distalen Endes des Seiles umfasst.

**3.** Verfahren nach Anspruch 1, wobei der Schritt zur Formung des Elektrodenleitungskörpers (110) ein Spritzgussverfahren umfasst, wobei Aussparung (111) und die erste und zweite Bohrung (112, 113) geformt werden.

**5.** Verfahren nach einem der Ansprüche 1 bis 3, wobei der Montageschritt folgende Schritte umfasst:
- Einfädeln des proximalen Endes (12b, 22b) der Zuleitung (12, 22) in die erste Bohrung (112) in oder an der zugeordneten Aussparung (111) und Schieben der Zuleitung in Richtung des proximalen Endes des Elektrodenleitungskörpers (110), bis die Elektrode (11, 21) in der Aussparung zu liegen kommt
- Kleben der Elektrode an den Elektrodenleitungskörper in der Aussparung
- elektrisches Verbinden des proximalen Endes der Zuleitung mit dem Stecker und Befestigen des Steckers am Elektrodenleitungskörper.
